# EUROPEAN PATENT APPLICATION

(11) **EP 1 785 897 A1**
(43) Date of publication of application: **16.05.2007**
(21) Application number: 05077541.0
(22) Date of filing: 04.11.2005
(51) Int. Cl.: G06F 19/00, C10M 125/00, G01L 3/00

(54) **Predictive technologies for lubricant development**

(71) Applicant: Avantium International B.V., 1014 BV Amsterdam (NL)
(72) Inventor: McKay, Benjamin, 1071 AT Amsterdam (NL)
(74) Representative: de Lang, Robbert-Jan

(57) **Abstract**

Method for predicting and/or correlating additive structure to engine performance, by developing molecular descriptors for one or more additives, developing a kinetic model for the engine performance using kinetic parameters, determine the relation between the molecular descriptors and the kinetic parameters by testing compositions comprising one or more additives, develop a QSPR library describing the relation between the molecular descriptors of the additives and the kinetic parameters and by predicting the engine performance of other additives using the QSPR library.

## Description

### FIELD OF THE INVENTION.

The present invention relates to methods for the efficient and high throughput development of lubricants for engines. The invention in particular relates to methods for predicting and/or correlating the structure of possible additives to lubricant and/or fuel performance in engines and to provide for methods that allow the prediction of lubricant and/or fuel performance in bench tests and/or engine tests and/or engines based on molecular descriptors derived from additives.

### BACKGROUND OF THE INVENTION.

In the lubricant industry, the drive toward zero emission, high energy efficiency engines demands new materials and innovative technologies to meet these goals. Another important driver for innovation is the increased environmental legislation for existing engines. New engine development is an expensive and time-consuming undertaking. Oftentimes, the long-term durability of the engine is controlled by the materials used, the mechanical design, and the effectiveness of the lubrication.

Historically, bench simulation tests are used to evaluate materials in combination with existing lubricants. Single-cylinder "bench tests" incorporating new materials or designs are conducted on an engine dynamometer to screen materials and designs. If successful, the materials and design are then tested in a prototype engine. If the material or design is significantly different from past experience, then a full-scale field test will be conducted to evaluate the long-term durability and material compatibility. Long-term lubricant-materials compatibility and durability issues often emerge at the last stage. This is expensive. Conversely, given an engine design, what kind of lubricant is needed to achieve satisfactory performance is difficult to ascertain at the beginning.

From the lubricant developers' perspective, since the new engine is evolving, many parameters have not been set and therefore it is difficult to define the lubricant needs for new engines. This leaves the lubricant developers with very little data to proceed with unitil the engine is sufficiently developed so that it can be made available for lubricant testing. At this time, however, many of the parameters including the materials have been fixed, and optimum lubrication may or may not be feasible.

An other issue is the warranty costs associated with the current production engines. The use of proper lubricants for a given engine design and operating conditions is vital in controlling the warranty costs. What lubricant is best for each engine operation and at what performance level the lubricant needs to be are issues the current engine manufacturers face. Current engine tests designed to qualify lubricants are becoming increasingly costly and complicated. Proper laboratory bench tests coupled with an analytical model can serve as a valuable aid to reduce the guesswork associated with the lubricant qualification testing.

An analytical model that is capable of predicting lubricant performance based on properties of lubricants and materials will significantly alleviate some of these issues.

To significantly reduce additive development timelines and reduce the number of laboratory bench tests and engine tests would be advantageous as they require the synthesis of large amount of additives to be tested and require prolonged and expensive testing. The reduction of the amount of bench tests, although in general cheaper and faster than engine tests, is also considered advantageous as they usually do not correlate very well (i.e. have a low predictive value) with the actual engine tests. Accordingly, there is a need to make better use of the experimental data and a need for faster and more reliable testing strategies using high throughput experimentation in combination with advanced design of experiments

### SUMMARY OF THE INVENTION

The present inventors have now found that experimentation, whether laboratory bench testing or engine testing, of lubricants and lubricant additives can significantly be reduced by developing a Quantitative Structure Performance Relationship (QSPR). In the QSPR, additive structures and lubricant formulation are correlated via a kinetic model and kinetic parameters and/or advanced engine simulation to additive and/or lubricant formulation performance. Additive structure as used herein refers to the chemical structure of a compound added to a lubricant of fuel formulation for the purpose of modifying the physiochemical properties of the formulation and thereby altering its performance in the engine or its stability in storage. In this way the QSPR can be used for prediction of novel additive and/or lubricant/fuel formulation performance. The additive structures are characterised by developing molecular descriptors. The correlation is achieved by determining the lubricant/fuel kinetic parameters directly from bench tests or by fitting kinetic parameters to the results of engine tests, development of lubricant/fuel and/or additive structure-property relationships (QSPRs) for predicting lubricant/fuel kinetic parameters, predicting kinetic parameters for novel additives in lubricant and/or fuel formulations and based on these predictions, simulating the performance in an actual engine. This typically involves modelling the lubricant.or fuel reactions in an engine or a part of the engine, determining the lubricant or fuel kinetics, simulating the lubricant or fuel performance in a laboratory bench test or in an.actual engine, testing the lubricant or fuel performance in a laboratory bench test or in an actual engine, developing lubricant/fuel and/or additive structure-kinetic relationships in a QSPR, and based on the relationships developed in the QSPR model and the engine simulation, predicting the performance of novel additives in lubricant/fuel formulations and/or, given a desired performance or modification in performance, predicting and designing the structure of.potential additives for lubricants and/or fuels.

### DETAILED DESCRIPTION OF THE INVENTION

Thus, the invention, in one aspect, relates to a method for predicting and/or correlating additive structure to engine performance comprising the steps of:
(a) providing a library of additives;
(b) characterising the additives in the library by molecular descriptors;
(c) providing an engine model, preferably comprising a set of interconnected reactor models;
(d) provide a kinetic model comprising kinetic parameters of reaction kinetics in an engine or engine model;
(e) formulating a plurality of compositions each comprising one or more additives from the library of (a);
(f) testing the compositions obtained under (d) and determining performance parameters;
(g) determine/fit the kinetic parameters in the model of (c) and/or (d) to the performance parameters for each composition tested under (e);
(h) providing a Quantitative Structure Performance Relationship (QSPR) model between the molecular descriptors of the additives in.the library of step (b) and the measured and/or fitted kinetic and/or performance parameters of step (f);
(i) predicting the performance of other additives based on the QSPR model of (g) and the engine model of (c) and/or (d).

Under predicting and/or correlating additive structure to engine performance is understood that, based on an initial set of data on the performance of characterised additives in a lubricant formulation, a relationship is developed between additive characteristics and the performance (emissions, deposit formation, wear) of an engine.

In step (a) a library of additives is provided. The additives in the library are characterised using a set of molecular descriptors. Typically each additive in the library is characterised using a set of molecular descriptors. Molecular descriptors in themselves are known, and are used to describe single molecular species properties. Typical molecular descriptors can be based on bulk physical properties such as boiling points, critical temperature, vapour pressure, flash point, auto-ignition temperature, density, refractive index, melting point, octanol-water partition coefficient, aqueous solubility of liquids and solids, molecular mass, water-air partition coefficients, GC retention times and response factor, critical micelle concentration, polymer glass transition temperature, polymer refractive index, and molecular descriptors such as partial charge and charge densities, dipole moment, molecular surface area, molecular volume, electrostatic potential, bond lengths, bond angels, heat of formation, hydrogen bonding ability, etc. See also Katritzky et al . Chem. Inf. Comput. Sci, 2000, 40, 1-18 and references cited therein on descriptor generation. Molecular descriptors can also include hash-key or structure fingerprints, constitutional descriptors such as functional group counts, fragment contribution, atomic contributions, topological descriptors such as connectivity indices, Wiener numbers and Balaban indices or geometric descriptors such as, solvent excluded volume and WHIM descriptors. In certain embodiments, typically in cases where novel compounds are to be designed and no bulk physical parameters are available, the generation of some molecular descriptors may require molecular modelling using, for example, molecular mechanics, semi-empirical or quantum mechanical methods.

In general, molecular descriptors provide a set of parameters that allow adequate description of changes in the molecular structure.

In step (c) of the method an engine model is provided. Preferably the engine model comprises a set of interconnected reactor models that model the whole engine or selected parts of the engine, such as the part where the most relevant chemical reactions are taking place. Examples thereof are the cylinder liner - piston ring interface, the oil sump, the top ring zone etc. Each part of the engine can be described as a separate chemical reactor, characterised by its own set of parameters as capacity, temperature, concentrations (reactant compositions), mass flow rates etc. The separate chemical reactors can be assembled to an interconnected set of reactors that represent the engine or a part thereof.

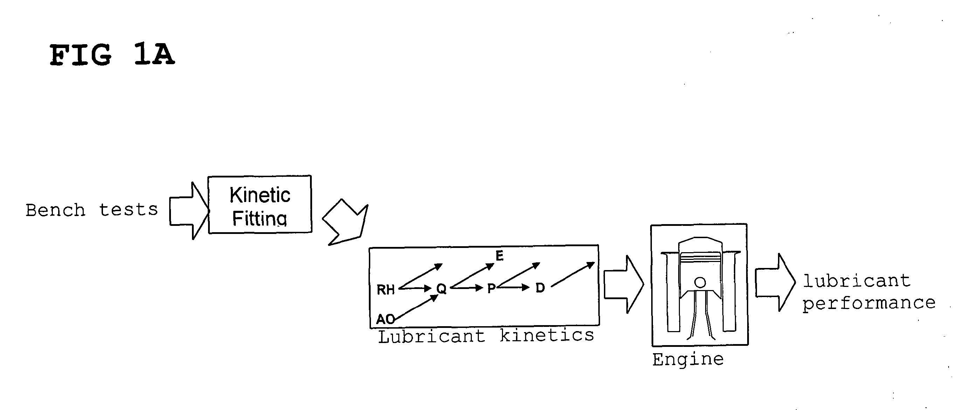

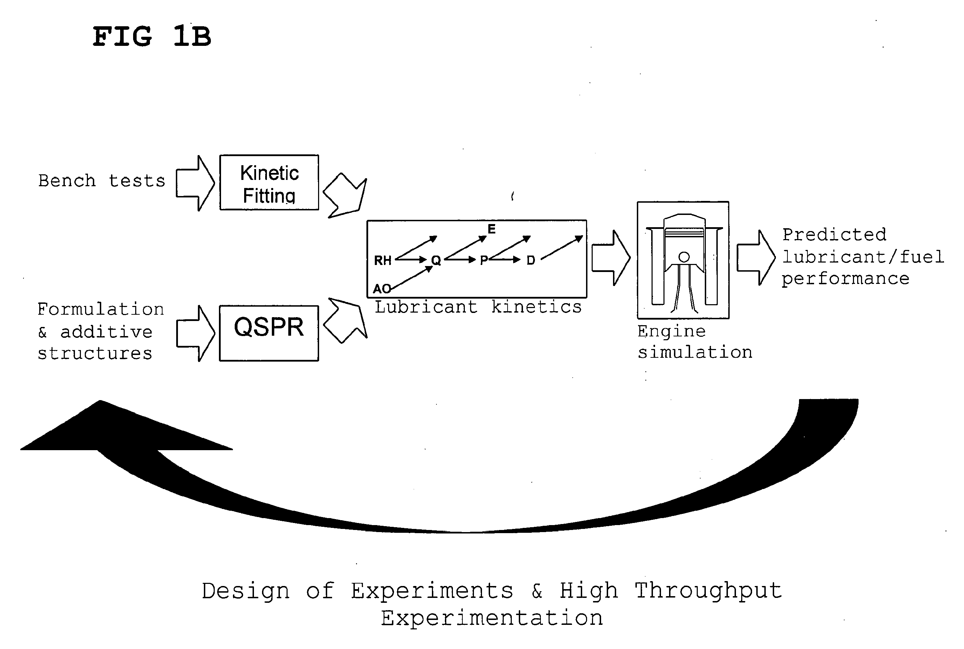

In step (d) of the method a kinetic model comprising kinetic parameters of reaction kinetics in an engine is provided. The model encompasses a reaction model comprising reaction rate equations and kinetic parameters. For each reactor model in the engine model such a set of equations can be developed. Such a model is depicted in Fig 1 for the oxidation and degradation of a lubricant comprising a lubricating component and an additive in an engine. For this model, the kinetics can be described with a set of rate equations:

Original Oil (RH) :

| | |
|---|---|
| dM(RH)/dt = -k1*M(RH)- k4*M(RH) | (Eq 1) |
| Primary Oxidation product (Q): dM(Q)/dt = k1*M(RH) + k8*M(AO)- k5*M(Q)-k2*M(Q) | (Eq 2) |
| High Molecular weight product (P): dM(P)/dt = k2*M(Q) - k3*M(P) - k6*M(P) | (Eq 3) |
| Deposit (D): dM(D)/dt = k3*M(P) - k7*M(D) | (Eq 4) |
| Evaporation (E): dM(E)/dt = k4*M(RH) + k5*M(Q) + k6*M(P) + k7*M(D) | (Eq 5) |
| Additive (AO) : dM(AO)/dt = - k8*M(AO) | (Eq 6) |

Thus, in certain embodiments, the rate equations for various individual components in the lubricant or fuel and the various reaction products produced can be selected from the group consisting of the fuel/lubricant rate equation, the additive rate equation, the evaporation rate equation, the deposit rate equation, the high Mw product formation rate equation, etc.

These reaction equations can be embedded in a multiple reactor system model described by a set of ordinary and differential equations. Such a reactor system can be configured to model an engine by choosing appropriate reactor temperatures, volumes, residence times and flow rates between the reactors. Such an approach to modelling a system of chemical reactors in itself well known in the reaction engineering literature. One example is for instance disclosed in the article of Chen et al. Tribology letters 1990, 14(2), 83 ff. albeit without any reference to apply this in an environment to predict performance based on QSPR modelling.

With the additives from the library, or at least a representative selection thereof, a plurality of compositions are formulated. Preferably the design of the compositions is such that the desired experimental space is covered, meaning that, based on for instance statistical analysis and cheminformatics, the compositions to be tested, are selected such that the set to be tested contains a representative number and variation of the different additives. The characteristics typically match the range of additives that are contemplated, not only for testing, but mainly for predicting in a later stage of the method. The plurality of compositions can also be determined using an automated or computerised design of experiments.

It is also possible to develop a combinatorial library of compositions to be tested, each member of the library comprising one or more additives in varying amounts. The plurality of compositions can be formulated in high throughput fashion using highly automated equipment such pippetting robots etc.

In step (e) of the method, the different compositions are tested under controlled circumstances, either in laboratory bench tests or in engine tests, depending on the circumstances, and the relevant performance parameters are determined. Typical performance parameters are wear, deposit formation, degradation, soot formation etc. A wide variety of technologies are known in the field and novel ones are developed everyday. Given the engine/kinetic model, suitable test procedures can be selected that provide for the rate constant in a reliable manner. As engines typically function over a range of temperatures, the Arrhenius equation can be used to accommodate for the change of the reaction constant as a function of the temperature. Possible technologies for evaporation under engine operating conditions can be thermal gravimetric analysis. Oxidation stability can be measured using DSC. For formation of high Mw products and deposit rate constants, a modified micro- oxidation test can be used according to Naidu et al. Ind. Eng. Chem. Prod. Res. Dev., 1986, 25, 596 etc.

In certain embodiments, the testing is, preferably simultaneously, performed in a plurality of laboratory bench tests and/or engine tests, preferably laboratory bench tests.

In step (f), the performance parameters are used to determine and/or to fit the kinetic parameters in the model such that the variation in the compositions and the effect on the performance is reflected in the determination of the kinetic parameters (the k's). In certain embodiments, the performance parameters are obtained by directly measuring the kinetic parameters in bench tests and/or engine tests..The kinetic parameters can also be obtained by kinetic fitting to the results of bench tests.

Preferably, the kinetic parameters are determined simultaneously, using high throughput methodologies such as kinetic fitting. This may involve numerically integrating a set of ordinary and differential equations describing the experiments and using an optimisation algorithm to select kinetic parameters that provide the best fit to the measured composition and performance parameters. An appropriate numerical integration algorithm may include a Runge-Kutta method, though preferably a method that is robust to stiff systems of equations will be used preferably an algorithm based on Gear's method.. Appropriate optimisation algorithms for the determination of the kinetic parameters include Gauss-Newton and Levenburg-Marquardt methods and includes solving the various rate equations discloses herein and determination of the k's for a variety of additives and formulations. Together with a statistical analysis.of the various rate constants for the various compositions, this provides a relation between the structure of the additive and the associated rate constants. The advantage of the fitting of the kinetic parameters resides in the ability to absorb poorly understood effects. It is clear from the above that the present method in itself is not limited to the set of rate equations exemplified herein, but that for a different set of equations, a similar approach can be applied.

Kinetic fitting of bench test data for a range of additive/lubricant compositions may be used to determine the.kinetic parameters for each of these compositions. Statistical analysis can then be used to develop a QSPR between the structures of the additive/lubricant components and in particular, one or more molecular descriptors of these additives/components. Performance in an engine (a laboratory test engine or actual engine) can then be determined by simulation in an engine model, for example by numerically integrating the ordinary and differential equations comprising the engine/kinetic model.

By solving the rate equations for the engine/kinetic model, a quantitative relation between the structure (of the additive/lubricant), and more in particular one or more of the the molecular descriptors, and the performance (or the kinetic parameters of the engine (laboratory test engine or actual engine) can be developed, resulting in a Quantitative Structure Performance Relationship (QSPR) model between the molecular descriptors of the additives in the library and the measured and/or fitted kinetic parameters. It will be clear that a Quantitative Structure Performance Relationship can also be a Quantitative Structure Kinetics Relationship (QSKR), depending on whether the performance parameters or the kinetic parameters are used in the development of the quantitative relation. It is noted that a QSPR or a QSKR is not conceptually different from a QSAR (Quantitative Structure Activity Relationship) such as is known from pharmaceutical fields. Such QSARs are described in the literature, and also for lubricant or fuel development, but not with respect to performance parameters or kinetic parameters.

Once a QSPR model is developed, typically based on a limited number of experiments or a limited set of available data, predictions can be made between potential additives.and their corresponding kinetic parameters and performance.in lubricant compositions. Also vice versa, when a certain performance of a lubricant or additive is desired, the corresponding performance parameter can be determined and, based on the associated kinetic parameters, the desired variation in molecular descriptors can be determined, leading to a number of suggestions on additive variations that based on the model may lead to the desired improvement of the performance parameter. These methods allow for the prediction of the effect of certain additives when these additives have not yet been actually tested, but have only been simulated in silico. Such an algorithm is described for instance in applicant's co-pending application EP1589463.

Thus, in certain embodiments, the performance of one or more other additives in a laboratory bench test and/or an engine test is predicted by predicting the kinetic parameters associated with the other additives using the QSPR and the engine/kinetic model. This can be achieved using an optimiser to find a set of kinetic parameters that meet a performance target. This means solving the inverse QSPR problem to find an additive structure that may give the desired kinetic parameters. One possible solution for this problem is described in EP1589463.

Preferably, the tests on which the QSPR is developed are laboratory bench tests. Nevertheless, once actual engine test data are obtained, the QSPR can be supplemented with these data to develop further and more accurate relations.

It is possible, for instance in the case of the availability of data that have been obtained in the scope of other experiments to create a so-called virtual library. These data from earlier tested additives can be coupled to molecular descriptors and the earlier test data can function as the performance parameters, thereby allowing the determination of the underlying kinetic parameters. These data can be used to help build the model and to further aid in developing a QSPR. Also, once predictions have been made for the performance or associated kinetic parameters for certain novel additives, the actual data once the novel additives have been tested can be supplemented to the QSPR, thereby further helping in developing the model and to improve the accuracy of the predictions. This supplementation of earlier or later obtained performance data, whether in the laboratory bench testing state or in the actual engine testing state, also aids in the improvement of the quality of the subsequent predictions, the same way as the development and incorporation of later developed molecular descriptors does.

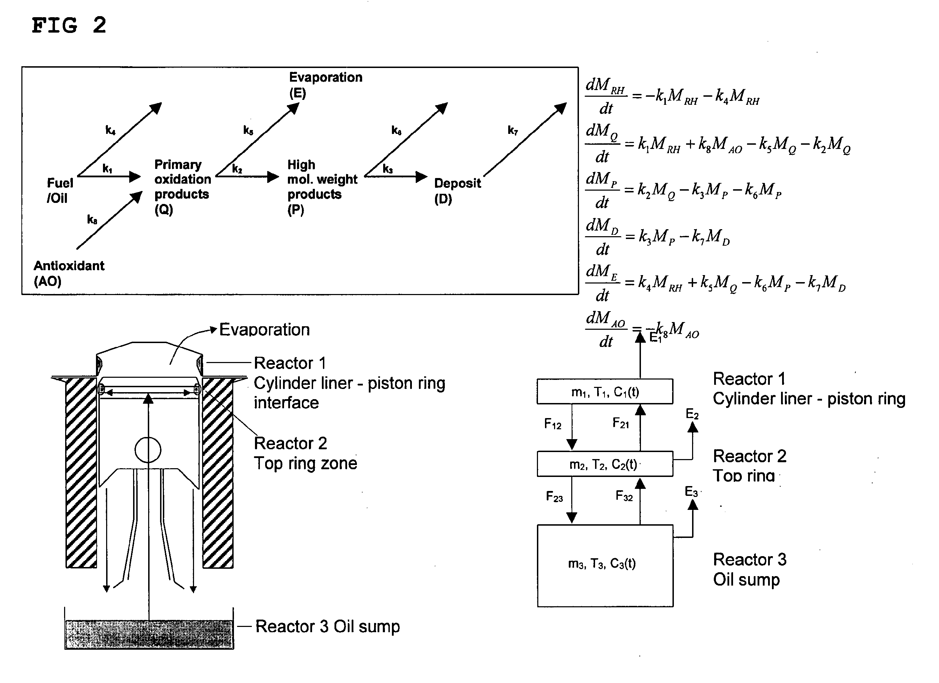

The prediction of suitable additives can be further improved by providing a model of the reactor (engine) and to provide a kinetic model that is capable of accommodating the various stages in the engine where the additive is taking its effect and the associated kinetics. The engine itself can be modelled in the form of a series of reactors as depicted in Fig 2.

A detailed description of the reaction kinetics of a complex mixture of lubricant with additives reacting under a wide range of reacting conditions is difficult. Simplification is preferred. Instead of treating individual species formation as a basis of reaction kinetic equation description, a broad class of reaction products can be used. Such a reaction model is shown in figure 2. The lubricant (RH) evaporates (k₄) and reacts with oxygen (k₁) to form the primary oxidation products (Q). The oxidised products also evaporate and further oxidise to form high-molecular weight products (P), which eventually form deposits (D). The extent of reactions is determined by the relative magnitudes of the rate constants (k's). Therefore, rate equations that describe the reaction system can be derived and are shown in the figure. When the rate constants can be independently determined, the resulting partial differential equations (with respect to time) can be solved numerically to provide the amount of each component at a given engine simulation point in time.

The engine is simulated by a number of reactors. The number of reactors in the system is determined by the engine design and usually matches the number of critical regions in the engine. Each region will define a set of environmental conditions for that reactor. See Fig. 2. The reactor is defined by the capacity of the reactor, the temperature, and the residence time. The characteristics of reactors are determined by the dimension of the engine and the actual operating conditions. The physical properties of the oils, such as viscosity, may also affect the capacity of the cylinder liner/piston ring interface (reactor) through variation in oil-film thickness. When the characteristics of a reactor and the lubricant are defined, simulation technology can be used to calculate the reaction products for a reactor as a function of time based on the kinetic equations outlined hereinbefore. Thus, for each reactor, s similar set of rate equations can be formulated. Other aspects of the engine operation such as oil flow rates among the reactors and the degree of mixing between the reactants and products in each reactor can also be taken into account or suitable assumptions, such as steady state flow rates and/or ideal mixing behaviour, can be made.

In certain embodiments, three reactors are used to represent the engine operation. The cylinder line/piston ring interface (reactor 1), the top ring groove and the volume above the ring (reactor 2) and the oil sump (reactor 3), as shown in the figure. In reactor 1, the average temperature per stroke cycle is very high (250-300°C) but the oxygen supply is usually limited due to combustion in the cylinder and the need for oxygen to diffuse into the oil film. Evaporation here is significant. In reactor 2, the residence time of the oil is much longer and the temperature is high (lower than reactor 1). With abundant oxygen, oxidative degradation reactions are rapid and deposits will accumulate. In the sump, the temperature is relatively low. The initiation step of oxidation is not likely to take place here. However, with the free radicals generated in the ring zone, a significant amount of sludge can form in the sump. The capacity (m), the average reaction temperature (T), and the residence time(t_{resd}) of each reactor are determined by specific engine designs. The concentration of a species C(t) at any time (t) is determined by the kinetics of the reactions.

The.oil-flow path is divided into two separate loops. Reactor 1 and reactor 2 constitute one loop. Reactor 2 and reactor 3 constitute another loop. This is to simulate the engine situation. The oil is first pumped from the oil sump (reactor 3) to the top piston ring zone (reactor 2), where the oil resides for several minutes. During this time, the oil circulates between the ring zone (reactor 2) and the cylinder liner/piston ring interface (reactor 1) continuously as the piston goes up and down. When the residence time of the oil in the ring zone (reactor 2) is reached, the oil leaves the ring zone and flows back to the sump (reactor 3). Each time the lubricant leaves a reactor, mass balance among the three reactors is performed to account for the evaporation and deposit accumulation, if any. The reacted lubricant then becomes the feed to another reactor and undergoes further reactions. The oil transport between reactors is governed by the residence times in each reactor. The :amount of oil exchanged will be determined by the oil pumping rate and mass balance under steady-state operation. Perfect mixing in each reactor is preferably assumed after oil exchange. The change in physical properties of the lubricant can be accommodated each time the mass balance is performed.

The necessary engine parameters needed such as the volume of the reactor, the temperature and residence time can be obtained either directly from the design of the engine (such as sump dimensions, oil pump rate, ring zone gap volume, or may need estimation or actual measurement.

Based on the above description, it is clear that, given the availability of a model of the kinetics in the engine, this development of a QSPR model based on kinetic data is applicable not only to lubricant additives, but is likewise applicable to fuel additives and fuel components.

In certain embodiments, the fuel is selected from the group consisting of motor fuels, preferably diesel fuel and/or gasoline, kerosene, jet fuels, marine bunker fuel, natural gas, home heating fuel and mixtures thereof.

In certain embodiments relating to fuel additives, the.fuel additive can be selected from the group consisting of detergents, cetane improvers, octane improvers, emission reducers, antioxidants, carrier fluids, metal deactivators, lead scavengers, rust inhibitors, bacteriostatic agents, corrosion inhibitors, antistatic additives, drag reducing agents, demulsifiers, dehazers, anti-icing additives, dispersants, combustion improvers and the like and mixtures thereof.

In certain embodiments relating to lubricant additives, the additives can be selected from the group consisting of antioxidants, anti-wear agents, detergents, rust inhibitors, dehazing agents, demulsifying agents, metal deactivating agents, friction modifiers, pour point depressants, antifoaming agents, co-solvents, package compatibilisers, corrosion-inhibitors, ashless dispersants, dyes, extreme pressure agents and mixtures thereof.

In certain embodiments, the method can be used for the screening of lubricant compositions comprising base oil and additives for compatibility with elastome.rs in terms of tensile strength measurement, selected from the group consisting of olefinic elastomers, styrenic elastomers, poly (ether/ester) elastomers, polyacrylate elastomers, natural rubbers, synthetic rubbers, elastomer seals and mixtures thereof.

In certain embodiments, the method can be used for determining deposit formation tendencies of additives and/ or for determining dispersancy performance in the additional presence of a lubricant insoluble material.

In certain embodiments, the method is performed under program control, i.e. software is used to carry out all or parts of the method, in particular relating to the flow of data, generation of molecular descriptors using calculational means such as modelling software, describing the kinetic model in terms of the rate equations and/or the engine model in terms of the interconnected model reactors, determination of the relation between the molecular descriptors and the kinetic parameters, the development of the QSPR model, the prediction of performance of novel additives based on the QSPR model and so on. Accordingly, the invention encompasses a computer program that, when it is run on a computer, is capable of exercising parts or all steps of the method.. From the detailed description of the method herein, the particular steps that can be performed by a computer program can be easily determined and accomplished. It is also understood that a library of additives as well as a set of molecular descriptors of each additive also means a set of data that can be used as input data in a computer program.

Typical examples of lubricants and additives are disclosed in WO2005/079277 WO2005/079278, WO2005/079279, WO2005/079280.

### DESCRIPTION OF THE FIGURES

**Fig 1A:** Schematic representation of the testing of an initial variety of additives in benchtests to fit the kinetic parameters of a kinetic model to accommodate the performance parameters in a model engine.
**Fig 1B:** Schematic representation of the prediction of lubricant performance based on an engine simulation using a QSPR comprising formulations and additive structure kinetic relationships and supplemented with possible additional data from further bench testing.
**Fig 2:** Schematic representation of a three reactor engine simulation, together with a kinetic model for lubricant additives. Reactor variables are m (capacity), T (average reaction temperature), t(resd) (residence time), concentration of a species C(t) at time t, F is the mass flow rate between reactors, E is evaporation from the reactor

## Claims

1. Method for predicting and/or correlating additive structure to engine performance, comprising the steps of:
(a) developing molecular descriptors for one or more additives;
(b) providing an engine model, preferably comprising a set of interconnected reactor models;
(c) provide a kinetic model comprising kinetic parameters of reaction kinetics in an engine or engine model;
(d) determining the relation between the molecular descriptors and the kinetic parameters by testing compositions comprising one or more additives;
(e) developing a QSPR model describing the relation between the molecular descriptors of the additives and the kinetic parameters;
(f) predicting the engine performance of other additives using the QSPR model and the engine simulation.

2. Method for predicting and/or correlating additive structure to engine performance comprising the steps of:
(a) providing a library of additives;
(b) characterising the additives in the library by molecular descriptors;
(c) providing an engine model, preferably comprising a set of interconnected reactor models;
(d) provide a kinetic model comprising kinetic parameters of reaction kinetics in an engine or engine model;
(e) formulating a plurality of compositions each comprising one or more additives from the library of (a);
(f) testing the compositions obtained under (e) and determine performance parameters;
(g) determine/fit the kinetic parameters in the model of (c)/(d) to the performance parameters for each composition tested under (e);
(h) providing a Quantitative Structure Performance Relationship (QSPR) model between the molecular descriptors of the additives in the library of step (b) and the measured and/or fitted kinetic parameters of step (f);
(i) predict the performance of other additives based on the QSPR model of (g) and the engine simulation.

3. Method according to claim 1 or 2, wherein the library is a actual library of additives, a combinatorial library, a virtual library or a combination thereof.

4. Method according to claims 1-3, wherein the additives are selected from the group consisting of lubricant additives, lubricant components, fuel additives, fuel components.

5. Method according to claim 4, wherein the fuel is selected from the group consisting of motor fuels, kerosene, jet fuels, marine bunker fuel, natural gas, home heating fuel and mixtures thereof.

6. Method according to claim 5, wherein the motor fuels are selected from the group consisting of diesel fuel and gasoline.

7. Method according to claim 4, wherein the at least one fuel additive is selected from the group consisting of detergents, cetane improvers, octane improvers, emission reducers, antioxidants, carrier fluids, metal deactivators, lead scavengers, rust inhibitors, bacteriostatic agents, corrosion inhibitors, antistatic additives, drag reducing agents, demulsifiers, dehazers, anti-icing additives, dispersants, combustion improvers and the like and mixtures thereof.

8. Method according to claim 4, wherein the lubricant additives are selected from the group consisting of antioxidants, anti-wear agents, detergents, rust inhibitors, dehazing agents, demulsifying agents, metal deactivating agents, friction modifiers, pour point depressants, antifoaming agents, co-solvents, package compatibilisers, corrosion-inhibitors, ashless dispersants, dyes, extreme pressure agents and mixtures thereof.

9. Method according to any of the previous claims, wherein the molecular descriptors are developed by a quantum mechanical approach, a molecular modelling approach, and combinations thereof.

10. Method according to any of the previous claims, wherein the molecular descriptors are selected from the group consisting of boiling point, critical temperature, vapour pressure, flash point, auto-ignition temperature, density, refractive index, melting point, octanol-water coefficient, fragment contribution, atomic contributions, partial charge and charge densities, dipole moment, molecular surface area, molecular volume, electrostatic potential, bond length, bond angel, heat of formation, hydrogen bonding ability, aqueous solubility of liquids and solids, molecular mass, water-air partition coefficient, GC retention time and response factor, critical micelle concentration, polymer glass transition temperature, polymer refractive index. hash-key or structure fingerprints, constitutional descriptors such as functional group counts, topological descriptors such as connectivity indices, Wiener numbers and Balaban indices or geometric descriptors such as molecular surface area, solvent excluded volume and WHIM descriptors

11. Method according to any of the previous claims, wherein the kinetic model is a model describing the lubricant degradation kinetics of an engine and/or the fuel combustion kinetics of an engine.

12. Method according to any of the previous claims, wherein the engine performance is a laboratory bench test, a laboratory bench test simulation, an engine test or an engine test simulation or a combination thereof.

13. Method according to any of the previous claims, wherein the plurality of compositions is formulated based on a combinatorial library.

14. Method according to any of the previous claims wherein the testing is performed in a high throughput environment.

15. Method according to any of the previous claims wherein the testing is, preferably simultaneously, performed in a plurality of laboratory bench tests and/or engine tests, preferably laboratory bench tests.

16. Method according to any of the previous claims, wherein the performance parameters are obtained from the laboratory bench tests and/or the engine tests.

17. Method according to any of the previous claims, wherein the QSPR model is developed based on laboratory testing and the predicted engine performance is an engine test.

18. Method according to any of the previous claims, wherein the performance parameters are obtained by directly measuring the kinetic parameters in bench tests or engine tests.

19. Method according to any of the previous claims, wherein the performance of other additives in a laboratory bench test and/or an engine test is predicted by predicting the kinetic parameters using the QSPR model.

20. Method according to any of the previous claims, wherein the kinetic parameters are rate constants.

21. Method according to any of the previous claims, wherein the kinetic model comprises rate equations.

22. Method according to any of the previous claims, wherein the engine is simulated by one or more of interconnected chemical reactors.

23. Method according to any of the previous claims, wherein each reactor is simulated by a kinetic model comprising rate equations.

24. Method according to any of the previous claims wherein the rate equations are the fuel/lubricant rate equation, the additive rate equation, the evaporation rate equation, the deposit rate equation, the high Mw product formation rate equation.

25. Use of a QSPR model for engine performance prediction of a fuel or lubricant additive.

26. Use according to claim 26, wherein the QSPR model is based on the fitting of kinetic parameters to performance parameters.

27. Computer program, comprising a set of instructions that, when running on a computer, perform the method as defined in any of the claims 1-25.
